(19) Europäisches Patentamt

European Patent Office

Office européen des brevets

(11) **EP 4 235 678 A1**

(12) **EUROPEAN PATENT APPLICATION**
published in accordance with Art. 153(4) EPC

(43) Date of publication:
**30.08.2023 Bulletin 2023/35**

(21) Application number: **20937176.4**

(22) Date of filing: **06.11.2020**

(51) International Patent Classification (IPC):
*G16B 30/10* (2019.01)    *G06F 13/42* (2006.01)
*G06F 9/50* (2006.01)

(52) Cooperative Patent Classification (CPC):
**Y02D 10/00**

(86) International application number:
**PCT/CN2020/127101**

(87) International publication number:
**WO 2022/082879 (28.04.2022 Gazette 2022/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.10.2020 CN 202011139823**

(71) Applicants:
• **BGI Genomics Co., Limited
Shenzhen, Guangdong 518000 (CN)**
• **Bgi Health (HK) Company Limited
Hong Kong 999077 (CN)**

(72) Inventors:
• **ZHANG, Youjin
Shenzhen, Guangdong 518000 (CN)**

• **YU, Chuang
Shenzhen, Guangdong 518000 (CN)**
• **KONG, Lingxiang
Shenzhen, Guangdong 518000 (CN)**
• **HE, Hui
Shenzhen, Guangdong 518000 (CN)**
• **HE, Zengquan
Shenzhen, Guangdong 518000 (CN)**
• **JIN, Xiangqian
Shenzhen, Guangdong 518000 (CN)**

(74) Representative: **Schwarz & Partner Patentanwälte
GmbH
Patentanwälte
Wipplingerstraße 30
1010 Wien (AT)**

(54) **GENE SEQUENCING DATA PROCESSING METHOD AND GENE SEQUENCING DATA PROCESSING DEVICE**

(57) The embodiments of the disclosure provide a method for processing gene sequencing data. The method is applied to an apparatus for processing gene sequencing data. The apparatus includes an advanced RISC machine, ARM, architecture, a graphics processing unit, GPU, architecture, and a peripheral component interconnect, PCI, bus; the ARM architecture includes at least one central processing unit, CPU, module; the GPU architecture includes at least one GPU module. The method includes: obtaining, by a CPU module in an idle state, batches of gene sequencing data by reading gene sequencing data in batches; obtaining, by the CPU module in the idle state, a first algorithm and a second algorithm by dividing a gene analyzing method; obtaining, by the CPU module in the idle state, a plurality of reads by dividing each batch of gene sequencing data based on the first algorithm, and sending the plurality of reads and the second algorithm to a GPU module in an idle state; performing, by the GPU module in the idle state, a calculation on each read based on the second algorithm, and sending respective calculation results to the CPU module in the idle state; and obtaining, by the CPU module in the idle state, a batch processing result based on the calculation results and the first algorithm. This method divides the analyzing method to run parts of the method on the CPU module and the GPU module, which greatly improves data analyzing efficiency.

EP 4 235 678 A1

**(Cont. next page)**

FIG. 1

## Description

This application claims priority to Chinese Patent Application No. 202011139823.4, filed on October 22, 2020, in China National Intellectual Property Administration, titled "Method for Processing Gene Sequencing Data and Apparatus for Processing Gene Sequencing Data", the entire content of which is incorporated herein by reference.

## TECHNICAL FIELD

The disclosure relates to a field of data processing technologies, and more particularly to a method for processing gene sequencing data and an apparatus for processing gene sequencing data.

## BACKGROUND

With continuous development of gene sequencing technology, the technology has been widely applied to research, development and analysis of new species, viruses and diseases. At the same time, a large amount of gene sequencing data has appeared, which makes it urgent to efficiently complete the analysis and processing of the large amount of gene sequencing data.

In the existing genetic analysis process (in the genetic alignment process), most of the steps can only be run on the x86 framework. For example, the traditional alignment algorithm bwa using the bwt algorithm and the Smith-Waterman of the imprecise alignment algorithm is also implemented based on the SSE2 instructions of the x86 architecture.

Although BWT alignment algorithm based on the x86 architecture runs fast on the CPU of the x86 architecture, the algorithm cannot calculate a large amount of data at the same time, and the BWT algorithm cannot adapt to the SIMT operation mode of the GPU, which greatly reduces the efficiency of BWT on the GPU, thereby affecting the efficiency of the entire alignment process. Similarly, the existing Smith-Waterman algorithm only runs on the x86 architecture, and lacks support of SSE2 acceleration on the ARM platform, and due to slow running speed, the algorithm is not suitable for computing on the GPU architecture.

## SUMMARY

In view of this, the disclosure provides a method for processing gene sequencing data and an apparatus for processing gene sequencing data, to solve a problem that the existing method for analyzing and processing the gene sequencing data can only run on the x86 architecture and run slowly on the GPU, which leads to low processing efficiency of the gene sequencing data.

Embodiments of the disclosure provide a method for processing gene sequencing data. The method is applied to an apparatus for processing gene sequencing data. The apparatus is a heterogeneous multi-core architecture, including an advanced RISC machine, ARM, architecture, a graphics processing unit, GPU, architecture, and a peripheral component interconnect, PCI, bus. The ARM architecture is connected to the GPU architecture through the PCI bus. The ARM architecture includes at least one central processing unit, CPU, module. The GPU architecture includes at least one GPU module. The method includes:

S1, obtaining, by a CPU module in an idle state, batches of gene sequencing data by reading gene sequencing data in batches;
S2, obtaining, by the CPU module in the idle state, a first algorithm and a second algorithm by dividing a gene analyzing method;
S3, obtaining, by the CPU module in the idle state, a plurality of reads by dividing each batch of gene sequencing data based on the first algorithm, and sending the plurality of reads and the second algorithm to a GPU module in an idle state;
S4, performing, by the GPU module in the idle state, a calculation on each read based on the second algorithm, and sending respective calculation results to the CPU module in the idle state;
S5, obtaining, by the CPU module in the idle state, a batch processing result based on the calculation results and the first algorithm; and
repeating S 1 to S5 until processing the gene sequencing data is completed, and obtaining, by the CPU module in the idle state, a final processing result by integrating the batch processing results.

Optionally, the CPU module in the idle state determines the number of the GPU modules in the idle state and a data processing amount corresponding to each GPU module in the idle state by scanning the at least one module, and reads the gene sequencing data in batches based on the number of the GPU modules in the idle state and the respective data processing amount.

Optionally, the gene analyzing method includes a gene alignment algorithm, a Dotplot algorithm, a blast algorithm, a partition around medoids, PAM, algorithm, a hidden Markov model, HMM, algorithm, and an artificial intelligence, AI, inference algorithm.

Optionally, the gene alignment algorithm includes a Burrows-Wheeler transform, BWT, algorithm, and the first algorithm includes an anchor point cutting algorithm; and the method further includes:

performing, by the CPU module in the idle state, an anchor point positioning operation on each batch of gene sequencing data through the anchor point cutting algorithm, and obtaining the plurality of reads by extending for a length of N $\times$ bp forward and backward respectively with the anchor point as a center and dividing each batch of gene sequencing data by a length of (2N+1) $\times$ bp based on a NEON instruction, where N is a positive integer.

Optionally, obtaining the plurality of reads includes:

obtaining the plurality of reads using a following formula:

$$(2*N+1)*x<L$$

where x represents the number of anchor points, N represents the number of extended bps, and L represents a length of each batch of gene sequencing data.

Optionally, the second algorithm is a Hash algorithm; and the method includes: preforming, by the GPU module in the idle state, a Hash operation on each of the plurality of reads based on the Hash algorithm to obtain a Hash calculation result, and sending the Hash calculation result to the CPU module in the idle state, in which the Hash calculation result is a value of a BWT algorithm matrix, used for calculation of the BWT algorithm matrix.

Optionally, the first algorithm further includes a BWT matrix transformation algorithm; and the method further includes: performing, by the CPU module in the idle state, a transformation operation on the BWT algorithm matrix based on the BWT matrix transformation algorithm, to obtain a BWT transformation result of the plurality of reads.

Optionally, the alignment algorithm includes a Smith-Waterman algorithm, and the second algorithm includes a scoring matrix algorithm. The method further includes: obtaining, by the GPU module in the idle state, a Smith-Waterman scoring matrix based on the scoring matrix algorithm, the plurality of reads, and a reference species sequence, and sending the Smith-Waterman scoring matrix to the CPU module in the idle state.

Optionally, obtaining the Smith-Waterman scoring matrix includes:

obtaining the Smith-Waterman scoring matrix using following formulas:

$$M=R*C$$

$$R=a*L^2+b$$

where, M represents the Smith-Waterman scoring matrix, R is a length of a candidate section of reference species sequence, C represents a length of a read generated by screening and splicing reads received from the CPU module in the idle state, L represents a length of each batch gene sequencing data, and a and b are constants.

Embodiments of the disclosure provide an apparatus for processing gene sequencing data, which is a heterogeneous multi-core framework. The apparatus is configured to execute the method for processing gene sequencing data.

With the apparatus for processing gene sequencing data and the method for processing gene sequencing data according to embodiments of the disclosure, the method is applied to the apparatus, and the apparatus is a heterogeneous multi-core architecture, including an ARM architecture, a GPU architecture, and a PCI bus. The ARM architecture includes at least one CPU module, and the GPU architecture includes at least one GPU module, and the CPU module is connected to the GPU module through the PCI bus to transfer information between each other. The CPU module in the idle state is mainly configured to read the gene sequencing data in batches and divide the gene analyzing method, to obtain batches of gene sequencing data, the first algorithm (this algorithm is the most suitable algorithm for the CPU module) and the second algorithm (this algorithm is the most suitable algorithm for the GPU module). Each batch of gene sequencing data is divided based on the first algorithm to obtain multiple reads, and the multiple reads and the second algorithm are sent to the GPU module in the idle state through the PCI bus. The GPU module performs a calculation on each read based on the second algorithm, and sends respective calculation results to the CPU module in the idle state. The CPU module in the idle state obtains a batch processing result based on the calculation results and the first algorithm. The CPU module in the idle state and the GPU module in the idle state repeat the above steps until processing the gene sequencing data is completed. The CPU module in the idle state obtains a final processing result by integrating the batch processing results. The apparatus for processing gene sequencing data and the method for processing gene sequencing data divide the analyzing method (i.e., analysis process) of gene sequencing data to run on the CPU module and GPU module separately according to the characteristics, which greatly improves the efficiency of analyzing the gene sequencing data. In addition, multiple CPU modules and GPU modules can be provided in the apparatus for processing gene sequencing data, and the multiple GPU modules can simultaneously calculate reads of different lengths, which can solve the problem of low parallel efficiency of GPU.

## BRIEF DESCRIPTION OF THE DRAWINGS

In order to clearly illustrate technical solutions of embodiments of the disclosure, a brief description of drawings used in embodiments is given below. Obviously, the drawings in the following descriptions are only part embodiments of the disclosure, and for those skilled in the art, other drawings can be obtained according to these drawings without creative labor.

FIG. 1 is a schematic diagram illustrating an apparatus for processing gene sequencing data according to embodiments of the disclosure.
FIG. 2 is a schematic diagram illustrating a data processing procedure of an apparatus for processing gene sequencing data according to embodiments of the disclosure.

FIG. 3 is a schematic diagram of performing an anchor point cutting operation on a batch of gene sequencing data by a CPU module according to embodiments of the disclosure.

FIG. 4 is a schematic diagram of performing a Hash operation based on a Hash algorithm by a GPU module according to embodiments of the disclosure.

FIG. 5 is a flowchart of a method for processing gene sequencing data according to embodiments of the disclosure.

## DETAILED DESCRIPTION

In order to enable those skilled in the art to understand the technical solutions of the disclosure, reference will be made clearly and completely technical solutions in the embodiments of the disclosure with accompanying drawings. Obviously, the embodiments described here are only part of the embodiments of the disclosure and are not all embodiments of the disclosure. Based on the embodiments of the disclosure, other embodiments obtained by those skilled in the art without creative labor are within scope of the disclosure.

## Explanation of nouns

Gene (Mendelian factor), it refers to a DNA or RNA sequence that carries genetic information (that is, gene is a DNA or RNA fragment with genetic effects), also known as a genetic factor, which is a basic genetic unit that controls traits. Gene expresses the genetic information by directing protein synthesis, to control phenotypic expression of biological identities.

Gene sequencing, it is a new type of gene detection technology that analyzes and determines a complete gene sequence from blood or saliva, to predict the possibility of suffering from a variety of diseases, individual behavior characteristics, and reasonable behavior.

Read, it is a short sequencing fragment, which is sequencing data generated by a highthroughput sequencer. Tens of millions of reads will be generated by sequencing the entire genome, and a complete gene sequence of the genome can be obtained by splicing these reads.

Alignment analysis, reads obtained after next generation sequencing (NGS) sequencing are stored in a FASTQ file. Although they are originally from an ordered genome, after establishing a DNA library and performing the gene sequencing, relative sequences between different reads in the file are lost. Therefore, there is no positional relationship between two closely adjacent reads in the FASTQ file and these two closely adjacent reads are randomly from two positions in the original genome. As a result, these reads need to be arranged in sequence first, compared with a reference genome of the same species one by one to find a corresponding position in the reference genome for each read, and finally arranged in order. The entire process is called the alignment of the sequencing data.

Alignment algorithm, there are generally two calculation methods for sequence alignment, one is global alignments and the other one is local alignments. A global route is calculated, which is a form of global optimization and forces all query sequences to be aligned with the entire length. In contrast, the local alignment only determines local similarity, which is very different from the entire long sequence. Local alignment is often desirable, but it is difficult to calculate due to challenges of identifying other similar regions. Various computational algorithms have been applied to solve sequence alignment problems, including slow but formal optimization methods such as dynamic programming, efficient but incomplete heuristic algorithms, or probabilistic methods for search design in large databases.

ARM or ARM architecture (Advanced RISC Machine, earlier known as Acorn RISC Machine), it is a RISC processor architecture family widely used in many embedded system designs. Due to its energy-saving characteristics, ARM also has a lot of contributions in other fields. ARM processor is very suitable for a field of mobile communication, in line with its main design goals of low cost, high performance, low power consumption characteristics. On the other hand, supercomputers consume a lot of power, and ARM is seen as a more efficient choice compared to supercomputers. ARM Holdings developed this architecture and authorized other companies to use this architecture to implement a certain architecture of ARM and develop their own system microcontroller unit (MCU) and system-on-module (SoC).

GUP, graphics processing unit (also known as display core, visual processor, display device or drawing device), it is a kind of microprocessor that specially runs graphics operations on personal computers, workstations, game consoles and mobile devices (such as tablets and smartphones). The GPU reduces dependence of the graphics card on the CPU, and shares part of the work originally performed by the CPU, and the effect is more obvious especially when performing three-dimensional drawing operations.

CUDA, compute unified device architecture, it is an integrated technology launched by NVIDIA, which is the official name for GPGPU. Through this technology, users can use the GPU after NVIDIA's GeForce 8 and new Quadro GPUs for calculations, which is the first time the GPU can be used as a development environment for C-compiler. In marketing, NVIDIA often promotes compilers mixed with architectures, which leads to confusion. In fact, CUDA is compatible with OpenCL or its own C-compiler. Whether it is CUDA C-language or OpenCL, the instructions will eventually be converted into PTX codes by the drive program and handed over to the display core for calculation.

BWT, Burrows-Wheeler Transform, it is an algorithm used in data compression technology, such as bzip2. The algorithm was proposed in 1994 by Michael Burrows and David Wheeler at the DEC systems research center in Palo Alto, California. The basis of this algorithm is an undisclosed conversion method developed by Wheeler in 1983. When a character string is converted by this algorithm, the algorithm only changes the order of the characters in the character string without changing the characters. If an original character string has several substrings that appear multiple times, there will be some consecutive repeated characters in the converted character string, which is very useful for compression. This method can make the encoding easier to be compressed based on the technology (such as MTF transformation and run length encoding) that deal with the continuous repeated characters in the character string.

Smith-Waterman, Smith-Waterman algorithm is an algorithm for local sequence alignment (relative to global alignment) to find similar regions between two nucleotide sequences or protein sequences. The purpose of this algorithm is not to perform the alignment of the complete sequence, but to find the fragments with high similarity between two sequences.

HASH, also known as Hash algorithm or Hash function, it is a method of creating small digital "fingerprints" from any kind of data. The Hash function compresses the message or data into a digest, so that the amount of data is reduced, and the format of the data is fixed. This function scrambles and mixes the data and recreates a fingerprint called hash value (hash code, hash sum, or hash). The hash value is generally represented by a short character string composed of random letters and numbers. A good hash function rarely has hash collisions in the input domain. In hash tables and data processing, distinguishing data without suppressing conflicts will make database records more difficult to find.

SSE2, Streaming SIMD Extensions 2, it is an IA-32 architecture single instruction multiple data (SIMD) instruction set. SSE2 is an instruction set introduced in 2001 along with the first generation of Pentium 4 processors released by Intel. SSE2 is an extension of the earlier SSE instruction set and can completely replace the MMX instruction set.

In order to explain the disclosure in detail, a method for processing gene sequencing data and an apparatus for processing gene sequencing data according to the disclosure will be described in detail below with reference to the accompanying drawings.

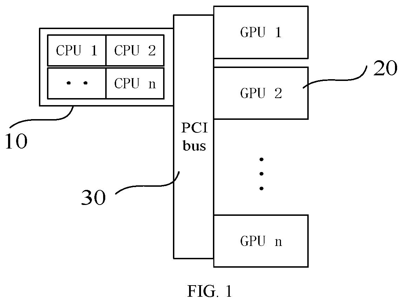

FIG. 1 is a schematic diagram illustrating an apparatus for processing gene sequencing data according to embodiments of the disclosure. As illustrated in FIG. 1, the apparatus is a heterogeneous multi-core architecture, including an ARM architecture 10, a GPU architecture 20, and a PCI bus 30. The ARM architecture 10 connects to the GPU architecture 20 through the PCI bus 30. The ARM architecture 10 includes at least one CPU module. The GPU architecture 20 includes at least one GPU module. The CPU module in the idle state is configured to obtain batches of gene sequencing data by reading gene sequencing data in batches, and obtain a first algorithm and a second algorithm by dividing a gene analyzing method. The CPU module in the idle state is configured to obtain reads by dividing each batch of gene sequencing data based on the first algorithm, and send the reads and the second algorithm to a GPU module in an idle state. The GPU module in the idle state is configured to perform a calculation on each read based on the second algorithm, and send the calculation result to the CPU module in the idle state. The CPU module in the idle state is further configured to obtain a batch processing result based on the calculation results and the first algorithm. The CPU module in the idle state and the GPU module in the idle state repeat the above processes until processing the gene sequencing data is completed, the CPU module in the idle state obtains a final processing result by integrating the batch processing results.

In detail, the apparatus for processing gene sequencing data is a heterogeneous multi-core architecture, that is, an ARM+GPU architecture. The ARM architecture 10 includes CPU modules, and the GPU architecture 20 includes GPU modules. The number of CPU modules and the number of GPU modules are not fixed, and can be set based on actual conditions, such as, based on the amount of gene sequencing data, CPU module performance, GPU module performance (e.g., GPU memory, CUDA core number, CUDA core frequency), and complexity of the gene analyzing algorithm.

The processing or computing capability of the core of each CPU module may be the same or different. Similarly, the processing or computing capability of each GPU module may also be the same or different. Optionally, the GPU module may be a GPU computing card, and the GPU computing card generally adopts the SIMT architecture.

In an optional implementation, the CPU module adopts NENO acceleration technology. The operating speed of the CPU module can be further improved by adopting this acceleration technology.

In an optional implementation, the apparatus for processing gene sequencing data can use the Jetson nano TX1 released by NVIDIA. The apparatus uses the GPU of a Maxwell architecture with 128 Cuda cores and a computing capability of 472G. In addition, Jetson-nano also has a 4-core A57 processor as the ARM CPU core operator.

Gene analyzing method refers to a method used in analyzing and processing the gene sequencing data, including sequence alignment, gene set enrichment analysis (including GO analysis and KEGG analysis), and gene regulatory network analysis.

The gene analyzing method is divided into the first algorithm and the second algorithm mainly based on the characteristics of the gene analyzing method. That is, the algorithm suitable for the CPU module is parted from the gene analyzing method as the first algorithm, the algorithm suitable for the GPU module is parted from the gene analyzing method as the second algorithm. It can be seen that the first algorithm and the second algorithm can be parts of the gene analyzing method, and can be consisted of one or more small steps. There are no strict algorithm rules in the dividing process as long as a dividing principle is met. The dividing principle mainly includes, the first algorithm generally requires a lot of logical judgments and there is a dependence between calculation results, for example, the calculation in a second step depends on or is based on a calculation result in a first step, or the judgment of yes or no is involved; and the second algorithm is generally that multiple data can be calculated in parallel and each data does not involve logical judgment or there is no dependence among the data.

It is to be understood that the "first" and "second" in embodiments are not limitations on the algorithm, but only to distinguish the two.

In addition, since there are multiple CPU modules in the ARM architecture 10, respective operation or working states of the CPU modules may be different, that is, some CPU modules are in a running state, and some are in an idle state. Similarly, the GPU modules in the GPU architecture 20 are in a similar case. Therefore, in embodiments, the CPU modules in the idle state and the GPU modules in the idle state are adopted to perform corresponding operations, where the selected CPU modules and GPU modules can be all modules in the idle state, or may be some of the modules in the idle state.

In addition, the gene sequencing data can be data obtained by performing gene sequencing of any species, including DNA sequencing fragments, RNA sequencing fragments, and the like. Since a large amount of data will be generated by performing the gene sequencing once, the data amount of the gene sequencing data will be relatively large. The data can be analyzed and processed in batches, thereby avoiding congestion of data transmission. Therefore, in embodiments, the CPU module in the idle state reads the gene sequencing data in batches. The number of pieces of gene sequencing data read each time may be unequal. In detail, the most suitable number of pieces of gene sequencing data can be determined by comprehensively considering the number of GPU modules, data processing capability of each GPU module, data reading capability of the CPU module, and data transmission capability of the PCI bus, to ensure the maximum data processing efficiency.

After reading the gene sequencing data in batches, it is generally necessary to divide each batch of gene sequencing data respectively to generate multiple reads. In embodiments, the first algorithm is used to divide each batch of gene sequencing data. The batch of gene sequencing data may be divided into reads having different lengths, and the number of the reads obtained by dividing each batch is not fixed, which can be determined by comprehensively considering the number of the batches of gene sequencing data, the number of GPU modules in the idle state, and the GPU processing capability.

After the CPU module in the idle state transmits each read and the second algorithm to the GPU in the idle state, the GPU module in the idle state performs a calculation on each read based on the second algorithm. At this time, the CPU module in the idle state can read and divide a next batch of gene sequencing data. When the GPU module in the idle state completes the processing of the reads, the calculation results are transmitted to the CPU module in the idle state. The CPU module can obtain a batch processing result based on the calculation results and the first algorithm. The above process is repeated continuously to form a pipeline between the CPU module and the GPU module until processing the gene sequencing data is completed.

With the apparatus for processing gene sequencing data according to embodiments of the disclosure, the apparatus is a heterogeneous multi-core architecture, including the ARM architecture 10, the GPU architecture 20 and the PCI bus 30. The ARM architecture includes at least one CPU module, and the GPU architecture includes at least one GPU module. The CPU module connects to the GPU module through the PCI bus to transfer information between each other. The CPU module in the idle state is mainly configured to read the gene sequencing data in batches and divide the gene analyzing method, to obtain batches of gene sequencing data, the first algorithm (this algorithm is the most suitable algorithm for the CPU module) and the second algorithm (this algorithm is the most suitable algorithm for the GPU module). The CPU module in the idle state is configured to divide each batch of gene sequencing data based on the first algorithm to obtain the reads, and send the reads and the second algorithm to the GPU module in the idle state through the PCI bus. The GPU module performs a calculation on each read based on the second algorithm, and send the calculation result to the CPU module in the idle state. The CPU module in the idle state obtains a batch processing result based on each calculation result and the first algorithm. The CPU module in the idle state and the GPU module in the idle state repeat the above steps until processing the gene sequencing data is completed, and the CPU module in the idle state obtains a final processing result by integrating each batch processing result. The apparatus for processing gene sequencing data and the method for processing gene sequencing data divide the analyzing method (i.e., analysis process) of gene sequencing data to run on the CPU module and GPU module separately according to the characteristics, which greatly improves the efficiency of analyzing the gene sequencing data. In addition, multiple CPU modules and GPU modules can be provided in the apparatus for processing gene sequencing data, and the multiple GPU modules can simultaneously perform calculation on reads of different lengths, which can solve a problem of low parallel efficiency of GPU.

In an embodiment, the CPU module in the idle state is also configured to scan each GPU module to determine the number of GPU modules in the idle state and the data processing capacity of each GPU module in the idle state, and read gene sequencing data in batches according to the number of GPU modules in the idle state and the data processing capacity of each GPU module.

In detail, when the gene analysis starts, the CPU module in the idle state scans the GPU modules to determine the number of currently available GPUs and the data processing capacity of each available GPU module, so as to determine the amount of gene sequencing data to be read in this batch, and read gene sequencing data based on the amount.

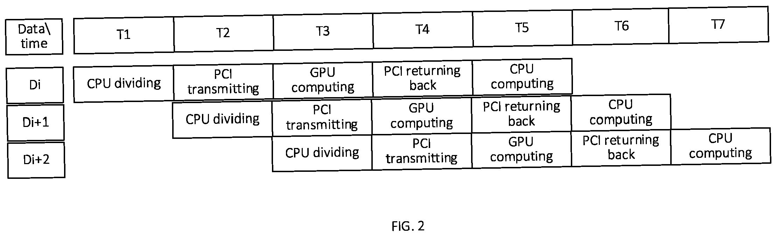

In order to facilitate the understanding of this solution, in combination with FIG. 1 and FIG. 2, a detailed embodiment of the pipeline of a method for processing gene sequencing data is given. In this embodiment, the gene analyzing method is a gene alignment method.

At the time of T1, the CPU module in the idle state receives gene sequencing data D, starts an alignment task program, and scans the number of currently available GPU modules. The number is labeled as G. The sequencing length of data D is recorded as L1. The CPU module reads the data D in batches. The amount of read data Di in each batch is recorded as K, where the value of K can be adjusted based on the number of GPU modules through a calculation formula K=A*G, where A represents the amount of data that the GPU module can process at one time (in this embodiment, the processing capability of each GPU module is selected to be exactly the same). The data Di is divided based on the first algorithm to generate multiple reads.

At the time of T2, the reads are transmitted to the GPU module in the idle state through the PCI bus, and at the same time, the CPU module can process a next batch of data Di+1, in order to generate a 2-stage pipeline.

At the time of T3, when the data Di is transmitted and arrives at a video memory of the GPU, GPU starts the second algorithm. At this time, Di+1 enters a PCI transmission stage, and the CPU module processes a next batch of data Di+2, to generate a 3-stage pipeline.

At the time of T4, after the calculation of data Di is completed, the calculation result is returned back to the CPU module through the PCI. At this time, the data Di+1 enters the GPU module calculation stage, the data Di+2 enters the PCI input stage, and the data Di+3 is processed by the CPU module to generate a 4-stage pipeline.

At the time of T5, after returning back the calculation result of the data Di is completed, the result is handed over to the CPU module to use the first algorithm to continue subsequent stages of the alignment algorithm to generate a 5-stage pipeline.

In an embodiment, the gene analyzing method includes a gene alignment algorithm, a Dotplot algorithm, a blast algorithm, a partition around medoids, PAM, algorithm, a hidden Markov model, HMM, algorithm, and an artificial intelligence, AI, inference algorithm.

In detail, the Dotplot algorithm and the blast algorithm are a kind of sequence alignment algorithm.

The PAM algorithm is a clustering algorithm for data mining, which can be used in singlecell sequencing to analyze cell subspecies.

The HMM algorithm, Hidden Markov Model, is a statistical model, which is used to describe a Markov process including hidden unknown parameters and can be used in the prediction of target genes.

The AI inference algorithm (DeepVariant) is a deep learning algorithm, which can be used to identify gene mutation. Optionally, the AI inference algorithm may be an inference algorithm related to a convolutional neural network (CNN) or a recurrent neural network (RNN).

Optionally, when the gene analyzing algorithm is the Dotplot algorithm, the blast algorithm or the PAM algorithm, it is usually necessary to perform CUDA on the algorithm first. Performing the CUDA on the algorithm makes the method more suitable to run on the apparatus for processing gene sequencing data according to embodiments of the disclosure.

In an embodiment, the gene alignment algorithm includes a BWT algorithm, and the first algorithm includes an anchor point cutting algorithm. The CPU module in the idle state is configured to perform an anchor point positioning operation on the batches of gene sequencing data through the anchor point cutting algorithm, and obtain the multiple reads by extending for a length of $N \times$ bp forward and backward respectively with the anchor point as a center and dividing each batch of gene sequencing data by a length of $(2N+1) \times$ bp based on a NEON instruction, where N is a positive integer.

In an embodiment, obtaining the multiple reads includes: obtaining the multiple reads using a following formula:

$$(2*N+1)*x < L$$

where x represents the number of anchor points, N represents the number of extended bps, and L represents a length of each batch of gene sequencing data.

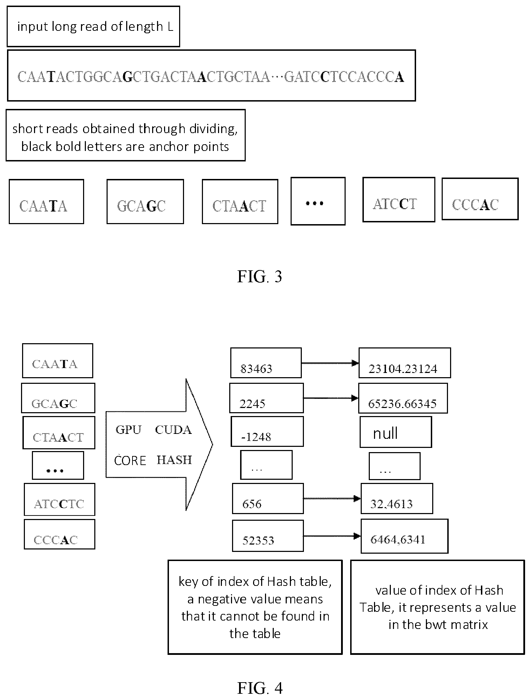

Optionally, the gene alignment algorithm may be a BWT algorithm, the first algorithm may be an anchor point cutting algorithm and a BWT matrix transformation algorithm, and the second algorithm is a Hash algorithm. As illustrated in FIG. 3, the CPU module in the idle state processes the data Di based on the first algorithm (i.e., the anchor point cutting algorithm). The CPU module in the idle state performs an anchor point positioning operation on each batch of gene sequencing data (reads) of a length L and obtains multiple short reads having a length of 2N+1 by extending for a length of $N \times$ bp forward and backward respectively, divides and transfers each batch of gene sequencing data by a length of 2N+1 based on a NEON instruction. When the number of anchor points is x, the number, i.e., N is related to a following formula:

$$(2*N+1)*x < L$$

where x represents the number of anchor points, N represents the number of extended bps, and L represents a length of each batch of gene sequencing data. The multiple reads can be obtained in the above-mentioned manner, which are suitable for running on the GPU module.

In an embodiment, the second algorithm is a Hash algorithm. The GPU module in the idle state performs a Hash operation on each of the multiple reads based on the Hash algorithm to obtain a Hash calculation result, and sends the Hash calculation result to the CPU module in the idle state. The Hash calculation result is a value of a BWT algorithm matrix, used for calculation of the BWT algorithm matrix.

In detail, the gene alignment algorithm may be a BWT algorithm, the first algorithm may be an anchor point cutting algorithm and a BWT matrix transformation algorithm, and the second algorithm may be a Hash algorithm. As illustrated in FIG. 4, x*K reads calculated based on the first algorithm are transferred to a video memory of the GPU module in the idle state, where K represents the number of pieces of data Di. The number of reads is positively correlated with the total video memory of the multiple GPU modules. Since the Hash algorithm is conducive to the operation of the SIMT architecture of the GPU module, a kernel function of the GPU is used to perform the Hash operation on the multiple reads to obtain the Hash calculation results, and the Hash calculation results are sent to the CPU module in the idle state. The Hash calculation results are values of the BWT algorithm matrix, used for calculation of the BWT algorithm matrix. Compared with other traditional calculations (such as kmer location calculation algorithm), the use of the Hash algorithm can greatly save the memory space.

In an embodiment, the first algorithm may be a BWT matrix transformation algorithm. The CPU module in the idle state is also configured to obtain the BWT transformation results of the reads by transferring the BWT algorithm matrix based on the BWT matrix transformation algorithm.

In detail, the gene alignment algorithm may be a BWT algorithm, and the first algorithm may be an anchor point cutting algorithm and a BWT matrix transformation algorithm. After the GPU module sends the Hash calculation results to the CPU module in the idle state, the CPU module performs a calculation operation on the BWT algorithm matrix by determining the Hash calculation results as the values of the BWT algorithm matrix, and performs a transformation operation on the BWT algorithm matrix based on the BWT matrix transformation algorithm, to obtain a BWT transformation result of the reads. Optionally, the relationship between the Hash calculation results and the BWT algorithm matrix is h=Hash(x,r), Y=BWT(h,r), where h represents the Hash calculation result, Y represents the BWT algorithm matrix, and r represents the read. This method can quickly and accurately obtain the BWT transformation result of the reads, thereby quickly completing the compression of the gene sequencing data, which is more convenient for subsequent processing.

In an embodiment, the alignment algorithm includes a Smith-Waterman algorithm, and the second algorithm includes a scoring matrix algorithm. The GPU module in the idle state calculates a Smith-Waterman scoring matrix based on the scoring matrix algorithm, the multiple reads, and a reference species sequence, and sends the Smith-Waterman scoring matrix to the CPU module in the idle state.

In an embodiment, obtaining the Smith-Waterman scoring matrix includes: obtaining the Smith-Waterman scoring matrix using following formulas:

$$M=R*C,$$
$$R=a*L^2+b$$

where, M represents the Smith-Waterman scoring matrix, R is a length of a candidate section of reference species sequence, C represents a length of a read generated by screening and splicing the multiple reads received from the CPU module in the idle state, L represents a length of each batch of gene sequencing data, and a and b are constants.

In detail, the traditional Smith-Waterman algorithm runs in the GPU with a low efficiency and cannot be directly used in the apparatus for processing gene sequencing data according to embodiments of the disclosure. Therefore, the Smith-Waterman algorithm is improved. In detail, there is a scoring matrix in the Smith-Waterman algorithm, and the size is R*C. The process of calculating the scoring matrix is executed by the GPU module, and the second algorithm is the scoring matrix algorithm at this time. The Smith-Waterman scoring matrix is obtained using following formulas:

$$M=R*C$$
$$R=a*L^2+b$$

where, M represents the Smith-Waterman scoring matrix, R is a length of a candidate section of reference species sequence, C represents a length of a read generated by screening and splicing multiple reads received from the CPU module in the idle state, L represents a length of the batch gene sequencing data, and a and b are constants. In addition, the length of C is related to the Hash calculation result calculated by the GPU module in the BWT algorithm. By adopting this method, the traditional Smith-Waterman algorithm can be improved to make it suitable for running in the GPU with a high operating efficiency.

According to the above-mentioned apparatus for processing gene sequencing data, embodiments of the disclosure also provide a method for processing gene sequencing data.

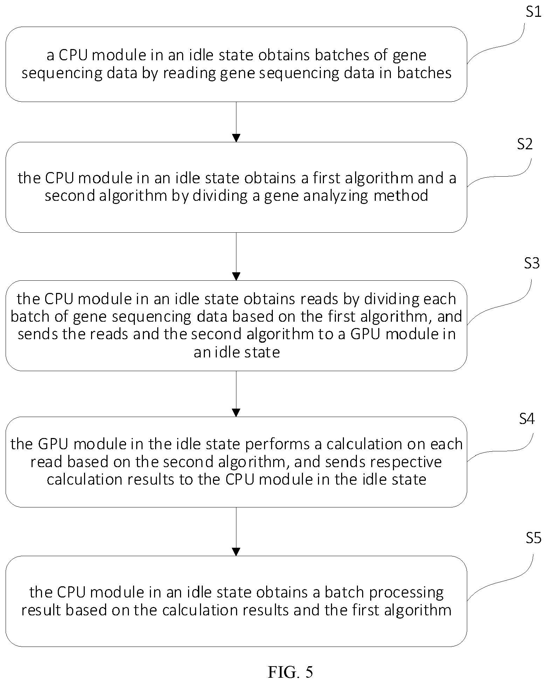

As illustrated in FIG. 5, the method for processing gene sequencing data is applied to the apparatus for processing gene sequencing data. The method includes the followings.

At block S1, the CPU module in an idle state obtains batches of gene sequencing data by reading gene sequencing data in batches.

At block S2, the CPU module in the idle state obtains a first algorithm and a second algorithm by dividing a gene analyzing method.

At block S3, the CPU module in the idle state obtains multiple reads by dividing each batch of gene sequencing data based on the first algorithm, and sends the multiple reads and the second algorithm to a GPU module in an idle state.

At block S4, the GPU module in the idle state performs a calculation on each read based on the second algorithm, and sending respective calculation results to the CPU module in the idle state.

At block S5, the CPU module in the idle state obtains a batch processing result based on the calculation results and the first algorithm.

The blocks S1 to S5 are repeated until processing the gene sequencing data is completed, and the CPU module in the idle state obtains a final processing result by integrating the batch processing results.

In detail, since a large amount of data is generated in one sequencing and the amount of the gene sequencing data is relatively large, the data can be analyzed and processed in batches, thereby avoiding the congestion of data transmission. The ith batch of gene sequencing data read by the CPU module in the idle state is recorded as Di. The CPU module in the idle state reads the gene sequencing data Di, and divides the gene analyzing method to obtain the first algorithm and the second algorithm. According to the first algorithm, the gene sequencing data Di is divided to into multiple reads, and the multiple reads and the second algorithm are sent to a GPU module in an idle state. The GPU module in the idle state performs a calculation on each read based on the second algorithm, and sends the calculation result to the CPU module in the idle state. The CPU module in the idle state obtains a batch processing result based on the calculation results and the first algorithm. In addition, the CPU module in the idle state reads the gene sequencing data Di+1, divides the gene sequencing data Di+1, and sends the reads obtained by dividing the gene sequencing data Di+1 to the GPU module in the idle state. Di+1 represents the read (i+1)th batch of gene sequencing data. The reads of the gene sequencing data Di+1 after being divided by the GPU module in the idle state are processed, and the processing results are sent to the CPU module in the idle state. The CPU module in the idle state and the GPU module in the idle state continue to read the gene sequencing data, divide, transmit, calculate and return (that is, repeating S1 to S5) until processing the gene sequencing data is completed. In this process, a pipeline is generated between the CPU module in the idle state and the GPU module in the idle state.

In an embodiment, the CPU module in the idle state scans each GPU module to determine the number of GPU modules in the idle state and the data processing capacity of each GPU module in the idle state, so as to read the gene sequencing data in batches based on the number of GPU modules in the idle state and the data processing capacity of each of the GPU modules in the idle state.

In an embodiment, the gene analyzing method includes a gene alignment algorithm, a Dotplot algorithm, a blast algorithm, a PAM algorithm, a HMM algorithm, and an AI inference algorithm.

In an embodiment, the gene alignment algorithm includes a BWT algorithm, and the first algorithm includes an anchor point cutting algorithm. The CPU module in the idle state performs an anchor point positioning operation on the batches of gene sequencing data through the anchor point cutting algorithm, and obtains the multiple reads by extending for a length of N × bp forward and backward respectively with the anchor point as a center and dividing each batch of gene sequencing data by a length of (2N+1) × bp based on a NEON instruction, where N is a positive integer.

In an embodiment, obtaining the multiple reads includes: obtaining the multiple reads using a following formula:

$$(2*N+1)*x < L$$

where x represents the number of anchor points, N represents the number of extended bps, and L represents a length of each batch of gene sequencing data.

In an embodiment, the second algorithm is a Hash algorithm. The GPU module in the idle state performs a Hash operation on each of the multiple reads based on the Hash algorithm to obtain a Hash calculation result, and sends the Hash calculation result to the CPU module in the idle state. The Hash calculation result is a value of a BWT algorithm matrix, used for calculation of the BWT algorithm matrix.

In an embodiment, the first algorithm further includes a BWT matrix transformation algorithm. The CPU module in the idle state is configured to perform a transformation operation on the BWT algorithm matrix based on the BWT matrix transformation algorithm, to obtain a BWT transformation result of the multiple reads.

In an embodiment, the alignment algorithm includes a Smith-Waterman algorithm, and the second algorithm includes a scoring matrix algorithm. The GPU module in the idle state is further configured to obtain a Smith-Waterman scoring matrix based on the scoring matrix algorithm, the multiple reads, and a reference species sequence, and send the Smith-Waterman scoring matrix to the CPU module in the idle state.

In an embodiment, obtaining the Smith-Waterman scoring matrix includes: obtaining the Smith-Waterman scoring matrix using following formulas:

$$M = R*C,$$

$$R = a*L^2 + b$$

where, M represents the Smith-Waterman scoring matrix, R is a length of a candidate section of reference species sequence, C represents a length of a read generated by screening and splicing the multiple reads received from the CPU module in the idle state, L represents a length of each batch of gene sequencing data, and a and b are constants.

Specific limitations of the method for processing gene sequencing data can refer to the above embodiments of the apparatus for processing gene sequencing data, which will not be repeated here.

Although explanatory embodiments have been shown and described, it would be appreciated by those skilled in the art that the above embodiments cannot be construed to limit the disclosure, and changes, alternatives, and modifications can be made in the embodiments without departing from scope of the disclosure.

**Claims**

1. A method for processing gene sequencing data, wherein the method is applied to an apparatus for processing gene sequencing data, the apparatus is a heterogeneous multi-core architecture, comprising an advanced RISC machine, ARM, architecture, a graphics processing unit, GPU, architecture, and a peripheral component interconnect, PCI, bus; the ARM architecture is connected to the GPU architecture through the PCI bus; the ARM architecture comprises at least one central processing unit, CPU, module; the GPU architecture comprises at least one GPU module; and the method comprises:

    S1, obtaining, by a CPU module in an idle state, batches of gene sequencing data by reading gene sequencing data in batches;
    S2, obtaining, by the CPU module in the idle state, a first algorithm and a second algorithm by dividing a gene analyzing method;
    S3, obtaining, by the CPU module in the idle state, a plurality of reads by dividing each batch of gene sequencing data based on the first algorithm, and sending the plurality of reads and the second algorithm to a GPU module in an idle state;
    S4, performing, by the GPU module in the idle state, a calculation on each read based on the second algorithm, and sending respective calculation results to the CPU module in the idle state;
    S5, obtaining, by the CPU module in the idle state, a batch processing result based on the calculation results and the first algorithm; and repeating S 1 to S5 until processing the gene sequencing data is completed, and obtaining, by the CPU module in the idle state, a final processing result by integrating the batch processing results.

2. The method according to claim 1, wherein the CPU module in the idle state determines the number of the GPU modules in the idle state and a data processing amount corresponding to each GPU module in the idle state by scanning the at least one module, and reads the gene sequencing data in batches based on the number of the GPU modules in the idle state and the respective data processing amount.

3. The method according to claim 1, wherein the gene analyzing method comprises a gene alignment algorithm, a Dotplot algorithm, a blast algorithm, a partition around medoids, PAM, algorithm, a hidden Markov model, HMM, algorithm, and an artificial intelligence, AI, inference algorithm.

4. The method according to claim 3, wherein the gene alignment algorithm comprises a Burrows-Wheeler transform, BWT, algorithm, and the first algorithm comprises an anchor point cutting algorithm; and the method further comprises:
performing, by the CPU module in the idle state, an anchor point positioning operation on each batch of gene sequencing data through the anchor point cutting algorithm, and obtaining the plurality of reads by extending for a length of N $\times$ bp forward and backward respectively with the anchor point as a center and dividing each batch of gene sequencing data by a length of (2N+1) $\times$ bp based on a NEON instruction, where N is a positive integer.

5. The method according to claim 4, wherein obtaining the plurality of reads comprises:
obtaining the plurality of reads using a following a formula:

$$(2*N+1)*x < L$$

where x represents the number of anchor points, N represents the number of extended bps, and L represents a length of each batch of gene sequencing data.

6. The method according to claim 3 or 4, wherein the second algorithm is a Hash algorithm; and the method comprises:
preforming, by the GPU module in the idle state, a Hash operation on each of the plurality of reads based on the Hash algorithm to obtain a Hash calculation result, and sending the Hash calculation result to the CPU module in the idle state, wherein the Hash calculation result is a value of a BWT algorithm matrix, used for calculation of the BWT algorithm matrix.

7. The method according to claim 6, wherein the first algorithm further comprises a BWT matrix transformation algorithm; and the method further comprises:
performing, by the CPU module in the idle state, a transformation operation on the BWT algorithm matrix based on the BWT matrix transformation algorithm, to obtain a BWT transformation result of the plurality of reads.

8. The method according to claim 3, wherein the alignment algorithm comprises a Smith-Waterman algorithm, and the second algorithm comprises a scoring matrix algorithm; and the method further comprises:
obtaining, by the GPU module in the idle state, a Smith-Waterman scoring matrix based on the scoring matrix algorithm, the plurality of reads, and a reference species sequence, and sending the Smith-Waterman scoring matrix to the CPU module in the idle state.

9. The method according to claim 8, wherein obtaining the Smith-Waterman scoring matrix comprises:
obtaining the Smith-Waterman scoring matrix using following formulas:

$$M=R*C$$

$$R=a*L^2+b$$

where, M represents the Smith-Waterman scoring matrix, R is a length of a candidate section of reference species sequence, C represents a length of a read generated by screening and splicing reads received from the CPU module in the idle state, L represents a length of each batch of gene sequencing data, and a and b are constants.

10. An apparatus for processing gene sequencing data, configured to execute a method for processing gene sequencing data according to any one of claims 1-9.

FIG. 1

| Data\time | T1 | T2 | T3 | T4 | T5 | T6 | T7 |
|---|---|---|---|---|---|---|---|
| Di | CPU dividing | PCI transmitting | GPU computing | PCI returning back | CPU computing | | |
| Di+1 | | CPU dividing | PCI transmitting | GPU computing | PCI returning back | CPU computing | |
| Di+2 | | | CPU dividing | PCI transmitting | GPU computing | PCI returning back | CPU computing |

FIG. 2

input long read of length L

CAATACTGGC**A**GCTGACTA**A**CTGCTAA···GATC**C**TCCACCC**A**

short reads obtained through dividing, black bold letters are anchor points

| CAATA | GCAGC | CTAACT | ··· | ATCCT | CCCAC |

FIG. 3

| CAATA |
| GCAGC |
| CTAACT |
| ··· |
| ATCCTC |
| CCCAC |

GPU CUDA CORE HASH

| 83463 | → | 23104.23124 |
| 2245 | → | 65236.66345 |
| -1248 | | null |
| ... | | ... |
| 656 | → | 32.4613 |
| 52353 | → | 6464.6341 |

key of index of Hash table, a negative value means that it cannot be found in the table

value of index of Hash Table, it represents a value in the bwt matrix

FIG. 4

S1

a CPU module in an idle state obtains batches of gene sequencing data by reading gene sequencing data in batches

S2

the CPU module in an idle state obtains a first algorithm and a second algorithm by dividing a gene analyzing method

S3

the CPU module in an idle state obtains reads by dividing each batch of gene sequencing data based on the first algorithm, and sends the reads and the second algorithm to a GPU module in an idle state

S4

the GPU module in the idle state performs a calculation on each read based on the second algorithm, and sends respective calculation results to the CPU module in the idle state

S5

the CPU module in an idle state obtains a batch processing result based on the calculation results and the first algorithm

FIG. 5

<table>
<tr><td colspan="2" align="center">**INTERNATIONAL SEARCH REPORT**</td><td colspan="2">International application No.<br>**PCT/CN2020/127101**</td></tr>
</table>

**A. CLASSIFICATION OF SUBJECT MATTER**

G16B 30/10(2019.01)i; G06F 13/42(2006.01)i; G06F 9/50(2006.01)i

According to International Patent Classification (IPC) or to both national classification and IPC

**B. FIELDS SEARCHED**

Minimum documentation searched (classification system followed by classification symbols)

G16B; G06F

Documentation searched other than minimum documentation to the extent that such documents are included in the fields searched

Electronic data base consulted during the international search (name of data base and, where practicable, search terms used)

CNPAT; CNKI; WPI; EPODOC; GOOGLE: 基因, 测序, 短序列, 分批, 异构, gene, sequenc+, PCI, CPU, ARM, GPU

**C. DOCUMENTS CONSIDERED TO BE RELEVANT**

| Category* | Citation of document, with indication, where appropriate, of the relevant passages | Relevant to claim No. |
|---|---|---|
| A | CN 104239732 A (HUNAN UNIVERSITY) 24 December 2014 (2014-12-24)<br>description, paragraph [0047] | 1-10 |
| A | CN 110473593 A (SHENZHEN UNIVERSITY) 19 November 2019 (2019-11-19)<br>entire document | 1-10 |
| A | CN 104504303 A (ZHAOQING UNIVERSITY) 08 April 2015 (2015-04-08)<br>entire document | 1-10 |
| A | CN 110135584 A (SOUTH CHINA UNIVERSITY OF TECHNOLOGY) 16 August 2019<br>(2019-08-16)<br>entire document | 1-10 |
| A | EP 3428798 A1 (HUAWEI TECHNOLOGIES CO., LTD.) 16 January 2019 (2019-01-16)<br>entire document | 1-10 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **07 July 2021** | **22 July 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2020/127101**

| Patent document cited in search report | | | Publication date (day/month/year) | Patent family member(s) | | | Publication date (day/month/year) |
|---|---|---|---|---|---|---|---|
| CN | 104239732 | A | 24 December 2014 | None | | | |
| CN | 110473593 | A | 19 November 2019 | None | | | |
| CN | 104504303 | A | 08 April 2015 | None | | | |
| CN | 110135584 | A | 16 August 2019 | None | | | |
| EP | 3428798 | A1 | 16 January 2019 | CN | 107273204 | A | 20 October 2017 |
| | | | | US | 2019042311 | A1 | 07 February 2019 |
| | | | | WO | 2017173968 | A1 | 12 October 2017 |

Form PCT/ISA/210 (patent family annex) (January 2015)

**REFERENCES CITED IN THE DESCRIPTION**

**Patent documents cited in the description**

- CN 202011139823